Europäisches Patentamt

European Patent Office

Office européen des brevets

(19)

(11) Numéro de publication: **0 194 390**
**A1**

(12) # DEMANDE DE BREVET EUROPEEN

(21) Numéro de dépôt: **85400468.6**

(22) Date de dépôt: **12.03.85**

(51) Int. Cl.⁴: **A 61 F 2/16**

(43) Date de publication de la demande:
**17.09.86 Bulletin 86/38**

(84) Etats contractants désignés:
**BE CH DE FR GB IT LI NL**

(71) Demandeur: **Dubois, Gérard**
**94, rue Haxo**
**F-75020 Paris(FR)**

(72) Inventeur: **Dubois, Gérard**
**94, rue Haxo**
**F-75020 Paris(FR)**

(74) Mandataire: **Sauvage, Renée**
**Cabinet Sauvage 100 bis, avenue de Saint-Mandé**
**F-75012 Paris(FR)**

(54) **Implants intra-oculaires à optique interchangeable.**

(57) L'invention porte sur une gamme, à combinaisons multiples, d'implants intra-oculaires formés, chacun, d'une part, d'une lentille correctrice et, d'autre part, d'une charpente de positionnement comportant un anneau d'enserrage dans lequel la lentille est susceptible de venir s'encliqueter, caractérisée en ce que la gamme comprend des lentilles (6) dans toutes les puissances correctrices couramment demandées et des charpentes de positionnement (1) dans toutes les dimensions et formes couramment utilisées, et en ce que, quelle que soit la puissance de la lentille (6) et quelles que soient la dimension et la forme de la charpente de positionnement (1):

l'anneau d'enserrage (4) a toujours le même diamètre interne D

les lentilles (6) ont toujours le mêmes diamètre D' égal au diamètre interne D de l'anneau d'enserrage (4)

la tranche interne de l'anneau d'enserrage (4) présente toujours le même relief d'encliquetage (5), et

le bord des lentilles (6) présente toujours le même relief d'encliquetage (7) complémentaire au relief (5) de l'anneau (4),

de sorte qu'il est possible de combiner n'importe quelle lentille (6) avec n'importe quelle charpente (1).

FIG 3

Implants intra-oculaires à optique interchangeable.

La présente invention concerne les implants intra-oculaires, c'est-à-dire les prothèses qui sont mises en place dans l'oeil après extraction du cristallin des sujets atteints de cataracte.

A l'heure actuelle, les implants intra-oculaires sont constitués d'une lentille munie d'anses de positionnement qui sont soit :
- solidarisées à la lentille, le plus souvent, par pénétration des anses dans la matière de la lentille et immobilisation en place, d'une manière ou d'une autre,
- soit réalisées d'une seule pièce, par usinage de l'ensemble monobloc.

Il a également été proposé (demande de brevet français n° 2.352.537 et demande de brevet allemande n° 2.710.272) d'encliqueter la lentille dans une charpente de positionnement présentant à cet effet une partie annulaire, mais à la connaissance du demandeur, cette technique n'a pas été utilisée en pratique.

Les implants d'une seule pièce sont quelquefois préférés par les chirurgiens du fait qu'il n'y a aucun risque de désolidarisation entre les anses de positionnement et la lentille.

Ces implants présentent cependant un sérieux inconvénient qui est lié aux multiples combinaisons possibles de puissances correctrices pour les lentilles et de formes et dimensions pour les anses ou charpentes de positionnement.

En effet, les puissances dépendent des besoins du patient, tandis que les formes et dimensions des charpentes sont fonction des cas particuliers d'implantation et du choix personnel du chirurgien.

Dès lors, le fabricant doit soit disposer d'un stock impressionnant de modèles qui constitue une immobilisation de fonds difficilement supportable, soit fabriquer les implants intra-oculaires à la demande, imposant ainsi un long délai de fourniture au chirurgien.

La présente invention a pour but de remédier à cette situation en proposant une gamme, à combinaisons multiples, d'implants intra-oculaires formés, chacun, d'une part d'une lentille correctrice et, d'autre part, d'une charpente de positionnement comportant un anneau d'enserrage dans lequel la lentille est susceptible de venir s'encliqueter, caractérisée en ce que la gamme comprend des lentilles dans toutes les puissances correctrices couramment demandées et des charpentes de positionnement dans toutes les dimensions et formes couramment utilisées, et en ce que, quelle que soit la puissance de la lentille et quelles que soient la dimension et la forme de la charpente de positionnement :

- l'anneau d'enserrage a toujours le même diamètre interne D
- les lentilles ont toujours le même diamètre D' égal au diamètre interne D de l'anneau d'enserrage
- la tranche interne de l'anneau d'enserrage présente toujours le même relief d'encliquetage, et
- le bord des lentilles présente toujours le même relief d'encliquetage complémentaire au relief de l'anneau, de sorte qu'il est possible de combiner n'importe quelle lentille avec n'importe quelle charpente.

L'encliquetage peut être obtenu par simple pression si l'anneau d'enserrage est suffisamment élastique. Sinon, l'encliquetage est réalisé soit en dilatant l'anneau d'enserrage, soit en rétractant la lentille, soit en combinant les deux opérations, en soumettant respectivement l'anneau et la lentille à des sources de chaleur ou de froid appropriées.

La lentille peut être plan-convexe, bi-convexe ou ménisque.

De cette manière, le fabricant peut fournir immédiatement un implant intra-oculaire satisfaisant aux divers paramètres requis par le chirurgien, sans être obligé de constituer un stock coûteux.

De plus, et surtout, l'invention apporte un progrès considérable pour le patient en ce sens qu'une fois l'implant intra-oculaire mis en place dans l'oeil, il peut être dissocié in situ par extraction de la lentille seule tandis que la charpente reste en place, puis l'implant peut être reconstitué par réencliquetage d'une autre lentille dans ladite charpente toujours in situ.

Jusqu'à présent, le remplacement de la lentille pouvant être nécessité soit par une correction impropre ou devenue insuffisante, soit par une détérioration de la lentille, notamment par suite de dépôts, demande l'extraction de l'intégralité de l'implant intra-oculaire et la remise en place d'un nouvel implant. Or, lorsqu'un implant intra-oculaire est en place dans l'oeil, les tissus se reforment autour des anses ou charpente de positionnement, tissus qu'il faut donc séparer pour pouvoir procéder à l'extraction. Grâce à l'invention, il devient possible de remplacer la lentille sans extraire la charpente, et donc de limiter au maximum l'agression à l'oeil du patient que constitue l'invervention chirurgicale.

Il va de soi que le matériau de la lentille et de la charpente doivent être inertes vis-à-vis de l'oeil. La lentille et la charpente pourront être faites du même matériau qui sera, de préférence, du poly(méthacrylate de méthyle).

D'autres caractéristiques de l'invention ressortiront de la description qui va suivre, faite en référence au dessin annexé.

Sur ce dessin :
- la figure 1 est une vue en plan d'une charpente de type KELMAN,
- la figure 2 est une coupe prise selon la ligne II-II de la figure 1,
- la figure 3 montre, à plus grande échelle, les zones coopérantes de la lentille et de l'anneau d'enserrage de la charpente,
- la figure 4 montre la même zone que la figure 3, après encliquetage de la lentille dans l'anneau d'enserrage,
- les figures 5a à 5e représentent schématiquement d'autres types de charpente, et
- les figures 6a à 6e représentent schématiquement divers types de lentilles susceptibles d'être encliquetées dans les divers types de charpente.

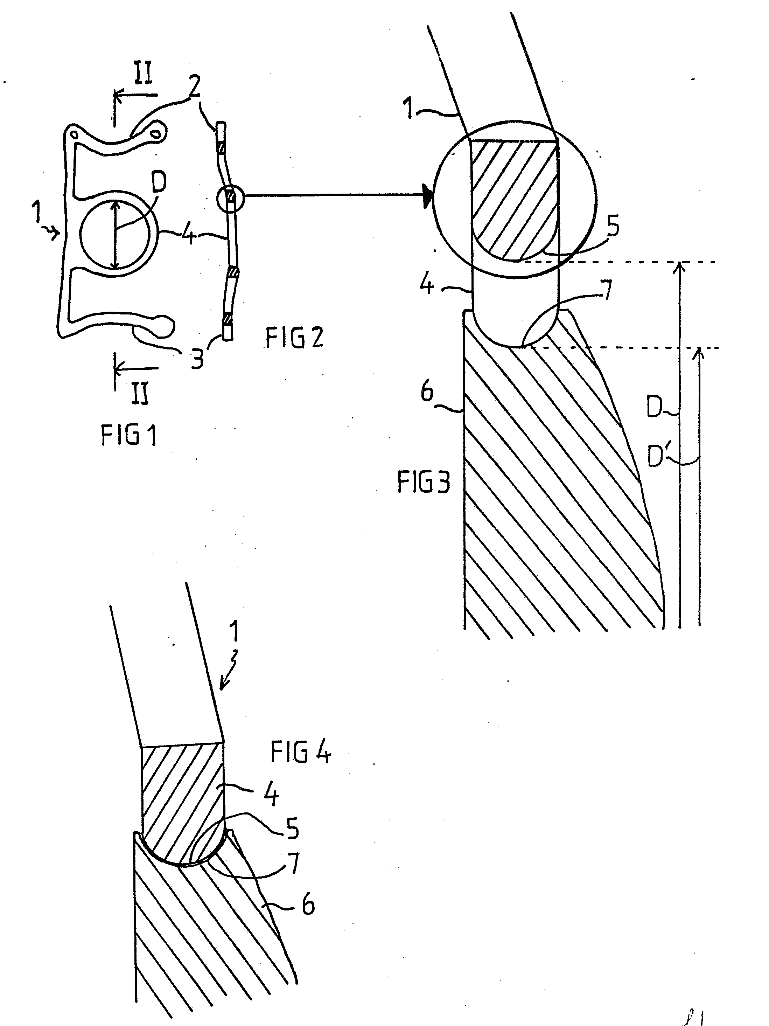

Si l'on se réfère aux figures 1 et 2, on voit une charpente de positionnement 1 comprenant des anses 2 et 3 et un anneau d'enserrage 4. Comme il ressort de la figure 3, la tranche intérieure de cet anneau 4 présente un relief convexe 5 soigneusement poli.

La figure 3 montre également une portion de lentille 6 dont le tranche présente une gorge ou relief concave 7, reproduction exacte, en creux, du relief 5 de l'anneau 4. Le diamètre $D$ de l'anneau 4, pris au sommet de son relief 5, est égal au diamètre $D'$ de la lentille pris au creux de son relief 7.

La lentille 6 est solidarisée de la charpente 1 par encliquetage dans l'anneau 4, avec pénétration du relief 5 dans le relief 7.

Le produit résultant, bien qu'en deux pièces, est aussi rigide et efficace que la lentille de KELMAN d'une pièce, elle est de meilleure qualité optique et beaucoup plus facile à fabriquer.

En effet, la lentille proprement dite est fabriquée par les procédés classiques éprouvés en optique de précision. De son côté, la charpente est polie au tonneau, comme le sont actuellement les implants intra-oculaires d'une seule pièce, mais sans risque de détérioration de la qualité de la partie optique puisqu'à ce stade, elle est absente.

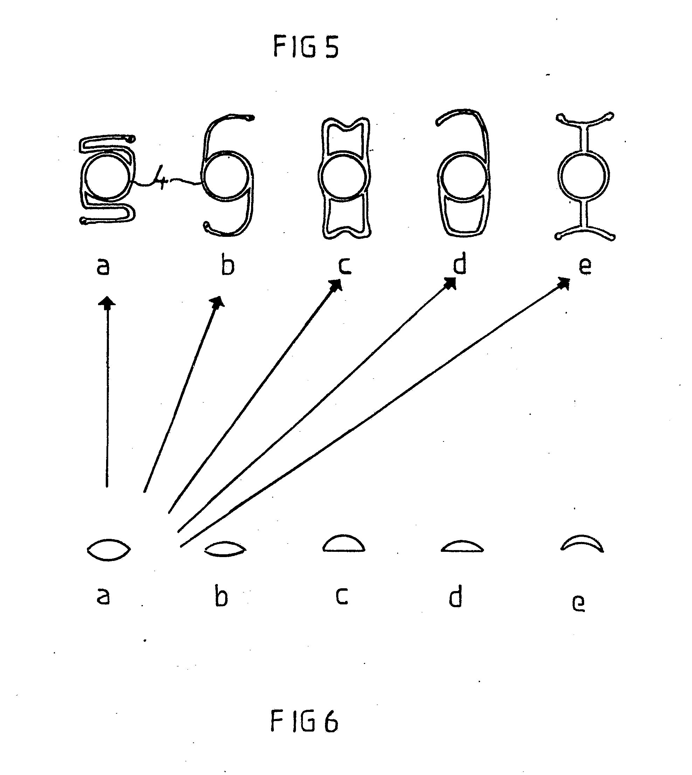

Les figures 5a à 5e montrent d'autres types de charpentes susceptibles d'entrer dans la gamme selon l'invention. Leur point commun est d'avoir toutes un anneau d'enserrage 4 de même diamètre interne et présentant un relief d'encliquetage qui est toujours le même.

Les figures 6a à 6e représentent divers types et puissances de lentilles : biconvexes (fig. 6a et 6b), plan-convexe (fig. 6c et 6d) et ménisque (fig. 6e). Leur point commun est d'avoir toutes le même diamètre et de présenter, sur leur bord, un relief d'encliquetage qui est toujours le même et complémentaire au relief des anneaux d'enserrage.

Comme le montrent les flèches, la lentille de la figure 6a est adaptée à être encliquetée sur l'une quelconque des charpentes des figures 5a à 5e. Il en va de même des

0194390

lentilles des figures 6b à 6e.

L'invention n'est évidemment pas limitée aux formes de charpentes représentées, mais est applicable à toute forme.

## REVENDICATIONS

1 - Gamme, à combinaisons multiples, d'implants intra-oculaires formés, chacun, d'une part, d'une lentille correctrice et, d'autre part, d'une charpente de positionnement comportant un anneau d'enserrage dans lequel la lentille est susceptible de venir s'encliqueter, caractérisée en ce que la gamme comprend des lentilles (6) dans toutes les puissances correctrices couramment demandées et des charpentes de positionnement (1) dans toutes les dimensions et formes couramment utilisées, et en ce que, quelle que soit la puissance de la lentille (6) et quelles que soient la dimension et la forme de la charpente de positionnement (1) :

- l'anneau d'enserrage (4) a toujours le même diamètre interne D
- les lentilles (6) ont toujours le même diamètre D' égal au diamètre interne D de l'anneau d'enserrage (4)
- la tranche interne de l'anneau d'enserrage (4) présente toujours le même relief d'encliquetage (5), et
- le bord des lentilles (6) présente toujours le même relief d'encliquetage (7) complémentaire au relief (5) de l'anneau (4),

de sorte qu'il est possible de combiner n'importe quelle lentille (6) avec n'importe quelle charpente (1).

2 - Gamme selon la revendication 1, caractérisée en ce que la lentille (6) est plan-convexe, bi-convexe ou ménisque.

3 - Gamme selon la revendication 1 ou 2, caractérisée en ce que les lentilles (6) et les charpentes (1) sont réalisées dans le même matériau inerte vis-à-vis de l'oeil.

4 - Gamme selon la revendication 3, caractérisée en ce que le matériau est du poly(méthacrylate de méthyle).

5 - Implant intra-oculaire appartenant à la gamme selon la revendication 1 ou 2, caractérisé en ce qu'une fois mis en place dans l'oeil, il peut être dissocié in situ par extraction de la lentille seule (6) tandis que la charpente (1) reste en place, puis l'implant peut être reconstitué par

0194390

réencliquetage d'une autre lentille (6) dans ladite charpente toujours in situ (1).

FIG 1
FIG 2
FIG 3
FIG 4

0194390

1/2

FIG 5

a        b        c        d        e

a        b        c        d        e

FIG 6

## DOCUMENTS CONSIDERES COMME PERTINENTS

**0194390**

| Catégorie | Citation du document avec indication, en cas de besoin, des parties pertinentes | Revendication concernée | CLASSEMENT DE LA DEMANDE (Int Cl 4) |
|---|---|---|---|
| X | US-A-3 925 825 (RICHARDS) <br> * Colonne 4, lignes 27-44; colonne 5, lignes 17-28; figures * | 1-5 | A 61 F  2/16 |
| D,X | FR-A-2 352 537 (POLER) <br> * Page 7, ligne 26 - page 8, ligne 24; figures 11-16 * | 1 | |
| D,X | DE-A-2 710 272 (KONSTANTINOV) <br> * Page 5, lignes 5-31; figures * | 1 | |
| E | FR-A-2 551 968 (DUBOIS) <br> * En entier * | 1-5 | |

-----

DOMAINES TECHNIQUES RECHERCHES (Int. Cl.4)

A 61 F

Le présent rapport de recherche a été établi pour toutes les revendications

| Lieu de la recherche | Date d'achèvement de la recherche | Examinateur |
|---|---|---|
| LA HAYE | 01-11-1985 | STEENBAKKER J. |